# EUROPEAN PATENT APPLICATION

(11) **EP 4 749 652 A1**
(43) Date of publication of application: **27.05.2026**
(21) Application number: 25217431.3
(22) Date of filing: 20.11.2025
(51) Int. Cl.: G16H 40/63, A61B 5/00, A61B 34/00

(54) **DISPLAY OF COMPLEX AND CONFLICTING INTERRELATED DATA STREAMS**

(30) Priority: 20.11.2024 US 202418954214
(71) Applicant: Cilag GmbH International, 6300 Zug (CH)
(72) Inventor: SHELTON IV, Frederick E., Cincinnati, 45242 (US); BRADY, John E., Cincinnati, 45242 (US); JONES, Shannon L., Cincinnati, 45242 (US); HARRIS, Jason L., Cincinnati, 45242 (US); COWPERTHWAIT, Matthew David, Cincinnati, 45242 (US); ROSS, Nicholas James, Cincinnati, 45242 (US); ADAMS, Shane R., Cincinnati, 45242 (US); HOLDMEYER, Seth, Cincinnati, 45242 (US)
(74) Representative: Carpmaels & Ransford LLP

(57) **Abstract**

Device and methods for displaying complex and conflicting interrelated data streams. An example device may receive a first biomarker value associated with a first biomarker in a first data stream and a second biomarker value associated with a second biomarker in a second data stream. The device may determine, based on the first biomarker value and the second biomarker value, that a close-loop control condition associated with a control parameter for the surgical device is failed. Based on determining that the close-loop control condition is failed, the device may identify an intraoperative metric associated with the first data stream and the second data stream. The device may generate a control signal configured to display a value associated with the intraoperative metric.

## Description

### Cross-Reference to Related Applications

This application claims the benefit of the following, the disclosures of which are incorporated herein by reference in its entirety:
- Provisional U.S. Patent Application No. 63/602,040, filed November 22, 2023
- Provisional U.S. Patent Application No. 63/602,028, filed November 22, 2023
- Provisional U.S. Patent Application No. 63/601,998, filed November 22, 2023
- Provisional U.S. Patent Application No. 63/602,003, filed November 22, 2023
- Provisional U.S. Patent Application No. 63/602,006, filed November 22, 2023
- Provisional U.S. Patent Application No. 63/602,011, filed November 22, 2023
- Provisional U.S. Patent Application No. 63/602,013, filed November 22, 2023,
- Provisional U.S. Patent Application No. 63/602,037, filed November 22, 2023, and
- Provisional U.S. Patent Application No. 63/602,007, filed November 22, 2023.

This application is related to the following, filed contemporaneously, the contents of each of which are incorporated by reference herein:
- U.S. Patent Application No. 18/954,186, filed November 20, 2024, entitled METHOD FOR MULTI-SYSTEM INTERACTION,
- U.S. Patent Application No. 18/954,195, filed November 20, 2024, entitled, VISUALIZATION OF AN INTERNAL PROCESS OF AN AUTOMATED OPERATION,
- U.S. Patent Application No. 18/954,199, filed November 20, 2024, entitled VISUALIZATION OF AUTOMATED SURGICAL SYSTEM DECISIONS,
- U.S. Patent Application No. 18/954,205, filed November 20, 2024, entitled VISUALIZATION OF EFFECTS OF DEVICE PLACEMENT IN AN OPERATING ROOM, and
- U.S. Patent Application No. 18/954,178, filed November 20, 2024, entitled VISUALIZATION OF EFFECTS OF DEVICE MOVEMENTS IN AN OPERATING ROOM.

### BACKGROUND

Surgical procedures are typically performed in surgical operating theaters or rooms in a healthcare facility such as, for example, a hospital. Various surgical devices and systems are utilized in performance of a surgical procedure. In the digital and information age, medical systems and facilities are often slower to implement systems or procedures utilizing newer and improved technologies due to patient safety and a general desire for maintaining traditional practices.

### SUMMARY

Devices and methods for displaying complex and conflicting interrelated data streams. An example device may include a processor configured to perform one or more actions. The device may receive a first biomarker value associated with a first biomarker in a first data stream and a second biomarker value associated with a second biomarker in a second data stream. The device may determine, based on the first biomarker value and the second biomarker value, that a close-loop control condition associated with a control parameter for a surgical device is satisfied. Based on determining that the close-loop control condition is satisfied, the device may determine a control parameter value associated with the surgical device based on the first biomarker value and the second biomarker value. The device may generate a control signal for the surgical device based on the determined control parameter value. The device may receive a third biomarker value associated with the first biomarker in the first data stream and a fourth biomarker value associated with the second biomarker in the second data stream. The device may determine, based on the third biomarker value and the fourth biomarker value, that the close-loop control condition associated with the control parameter for the surgical device is failed. Based on determining that the close-loop control condition is failed, the device may identify an intraoperative metric associated with the first data stream and the second data stream. The device may generate a second control signal configured to display a value associated with the intraoperative metric.

The first biomarker and the second biomarker may be associated with a physiological function of a patient. The device may determine a first status of the physiological function based on the first biomarker value and the second biomarker value. The close-loop control condition may be determined to be satisfied based on the first status of the physiological function being within an expected range. The device may determine a second status of the physiological function based on the third biomarker value and the fourth biomarker value. The close-loop control condition may be determined to be failed based on the second status of the physiological function being outside the expected range.

The device may determine a status type of the second status, wherein the status type indicates at least one of: at least one of the first biomarker or the second biomarker has changed at a rate that is greater than a first threshold, at least one of the first biomarker or the second biomarker has fluctuated a number of times during a time window, wherein the number of times is greater than a second threshold, a difference between the first biomarker and the second biomarker is greater than a third threshold, or a timing delay between a change in the first data stream and a change in the second data stream is greater than a fourth threshold. The intraoperative metric may be identified based on the status type of the second status.

The first biomarker and the second biomarker may be associated with a physiological function of a patient. The device may identify a third biomarker associated with the physiological function of the patient. The device may determine that the third biomarker is capable of impacting at least one of the first biomarker or the second biomarker. Based on the determination that the third biomarker is capable of impacting at least one of the first biomarker or the second biomarker, the device may use the third biomarker as the intraoperative metric.

The device may determine a first control parameter change direction associated with the control parameter based on the first biomarker value. The device may determine a second control parameter change direction associated with control parameter based on the second biomarker value. The close-loop control condition may be determined to be satisfied based on the first control parameter change direction and the second control parameter change direction being the same. The device may determine a third control parameter change direction associated with the control parameter based on the third biomarker value. The device may determine a fourth control parameter change direction associated with the control parameter based on the fourth biomarker value. The close-loop control condition may be determined to be failed based on the third control parameter changing direction and the fourth control parameter changing direction being different.

The device may determine a correlation pattern of the first data stream and the second data stream. The close-loop control condition may be determined to be satisfied or failed based on the correlation pattern.

The first biomarker may be a blood oxygen content. The second biomarker may be a percentage of carbon dioxide in exhalations. The device may determine a correlation pattern of blood oxygen content measurements in the first data stream and percentage of carbon dioxide in exhalations measurements in the second data stream. The close-loop control condition may be determined to be satisfied based on the correlation pattern indicating that the percentage of carbon dioxide in exhalations measurements and the blood oxygen content measurements change at a same rate. The device may generate a visual indication of a slope comparison of the first data stream and the second data stream. The intraoperative metric may include the slope comparison of the first data stream and the second data stream.

The first biomarker may be a blood oxygen content. The second biomarker may be a percentage of carbon dioxide in exhalations. The device may determine a correlation pattern of blood oxygen content measurements in the first data stream and percentage of carbon dioxide in exhalations measurements in the second data stream. The close-loop control condition may be determined to be failed based on the correlation pattern indicating that the percentage of carbon dioxide in exhalations measurements and the blood oxygen content measurements drift apart. Based on determining that the percentage of carbon dioxide in exhalations measurements and the blood oxygen content measurements drift apart, the device may identify a core body temperature of a patient as the intraoperative metric for display.

The device may determine a first pattern of the first data stream. The device may determine a second pattern of the second data stream. The intraoperative metric may include the first pattern of the first data stream and the second pattern of the second data stream.

The device may determine a timing delay between a change in the first data stream and a change in the second data stream. The intraoperative metric may include the determined timing delay between the change in the first data stream and the change in the second data stream.

The device may determine that the first data stream has stopped being received. Based on determining that the first data stream has stopped, the device may include, in the intraoperative metric comprises an option to use simulated data based on a pattern of the first biomarker while the first data stream was being received.

The device may determine a format of a graphical representation of the first data stream and the second data stream based on the intraoperative metric. The device may generate the graphical representation based on the determined format. The second control signal may be configured to instruct a display to display the generated graphical representation.

The second control signal may indicate a prompt or suggestion. The device may receive an input in response to the prompt or suggestion. The device may generate a third control signal for the surgical device based on received response.

### BRIEF DESCRIPTION OF THE DRAWINGS

FIG. 1 is a block diagram of a computer-implemented surgical system.
FIG. 2 illustrates an example surgical system in a surgical operating room.
FIG. 3 illustrates an example surgical hub paired with various systems.
FIG. 4 illustrates an example situationally aware surgical system.
FIG. 5 illustrates an example operating room arrangement of multiple robotic devices.
FIGs. 6A-C illustrate example conflicting decisions based on data from a systemic warming device and data from a smart ventilator.
FIGs. 7A-B illustrate example conflicting decisions based on data from a smoke evacuator, generator, and surgical scope.
FIGs. 8A-B illustrate example threshold options.
FIGs. 9A-B illustrate an example of highlighting segments of high variability.
FIGs. 10A-B illustrate example options for displaying historical data.

### DETAILED DESCRIPTION

A more detailed understanding may be had from the following description, given by way of example in conjunction with the accompanying drawings.

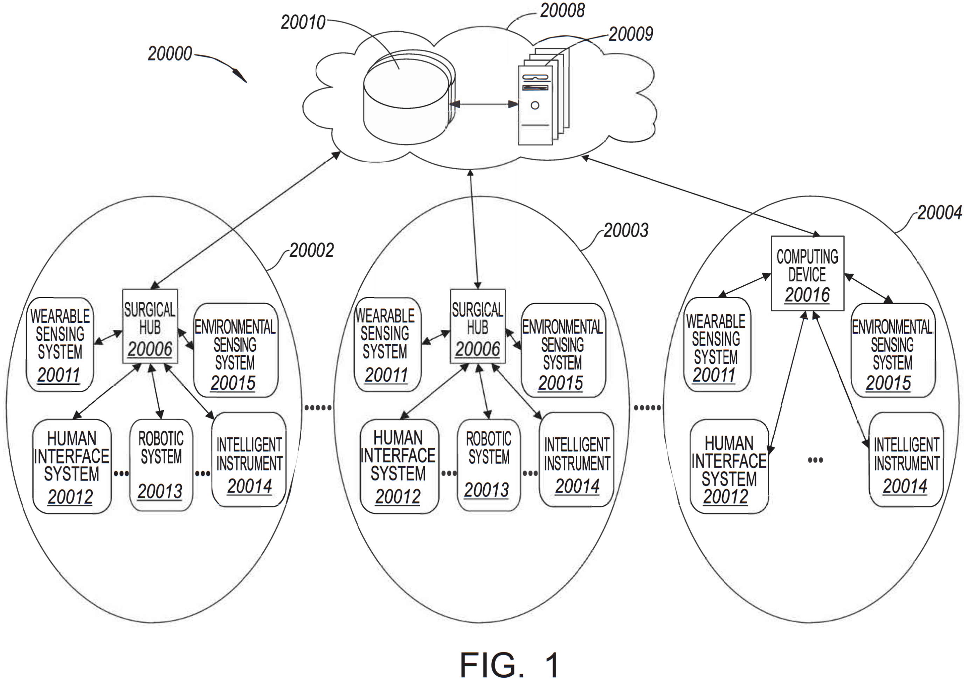

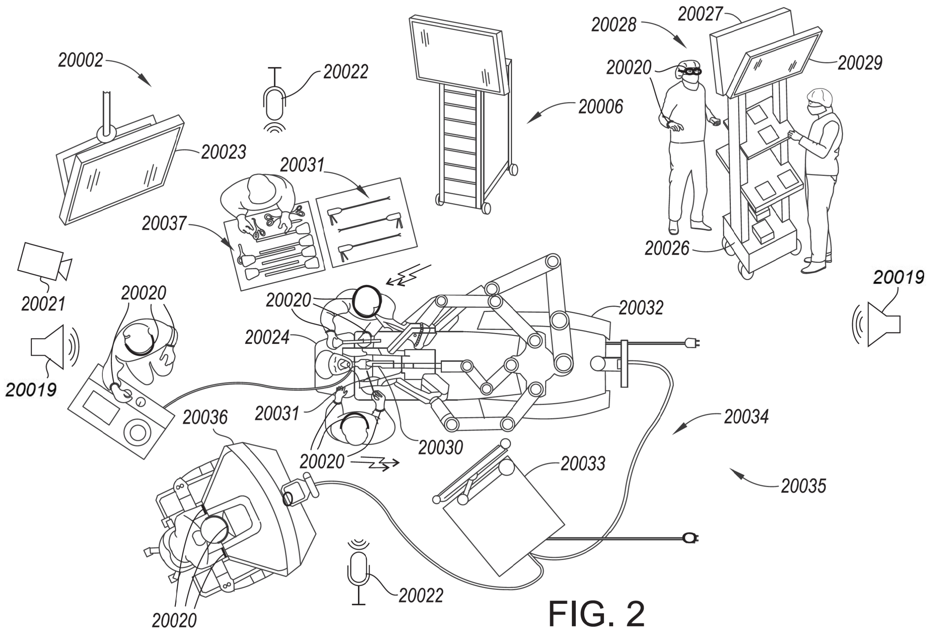

FIG. 1 shows an example computer-implemented surgical system 20000. The example surgical system 20000 may include one or more surgical systems (e.g., surgical sub-systems) 20002, 20003 and 20004. For example, surgical system 20002 may include a computer-implemented interactive surgical system. For example, surgical system 20002 may include a surgical hub 20006 and/or a computing device 20016 in communication with a cloud computing system 20008, for example, as described in FIG. 2. The cloud computing system 20008 may include at least one remote cloud server 20009 and at least one remote cloud storage unit 20010. Example surgical systems 20002, 20003, or 20004 may include one or more wearable sensing systems 20011, one or more environmental sensing systems 20015, one or more robotic systems 20013, one or more intelligent instruments 20014, one or more human interface systems 20012, etc. The human interface system is also referred herein as the human interface device. The wearable sensing system 20011 may include one or more health care professional (HCP) sensing systems, and/or one or more patient sensing systems. The environmental sensing system 20015 may include one or more devices, for example, used for measuring one or more environmental attributes, for example, as further described in FIG. 2. The robotic system 20013 may include a plurality of devices used for performing a surgical procedure, for example, as further described in FIG. 2.

The surgical system 20002 may be in communication with a remote server 20009 that may be part of a cloud computing system 20008. In an example, the surgical system 20002 may be in communication with a remote server 20009 via an internet service provider's cable/FIOS networking node. In an example, a patient sensing system may be in direct communication with a remote server 20009. The surgical system 20002 (and/or various sub-systems, smart surgical instruments, robots, sensing systems, and other computerized devices described herein) may collect data in real-time and transfer the data to cloud computers for data processing and manipulation. It will be appreciated that cloud computing may rely on sharing computing resources rather than having local servers or personal devices to handle software applications.

The surgical system 20002 and/or a component therein may communicate with the remote servers 20009 via a cellular transmission/reception point (TRP) or a base station using one or more of the following cellular protocols: GSM/GPRS/EDGE (2G), UMTS/HSPA (3G), long term evolution (LTE) or 4G, LTE-Advanced (LTE-A), new radio (NR) or 5G, and/or other wired or wireless communication protocols. Various examples of cloud-based analytics that are performed by the cloud computing system 20008, and are suitable for use with the present disclosure, are described in U.S. Patent Application Publication No. US 2019-0206569 A1 (U.S. Patent Application No. 16/209,403), titled METHOD OF CLOUD BASED DATA ANALYTICS FOR USE WITH THE HUB, filed December 4, 2018, the disclosure of which is herein incorporated by reference in its entirety.

The surgical hub 20006 may have cooperative interactions with one of more means of displaying the image from the laparoscopic scope and information from one or more other smart devices and one or more sensing systems 20011. The surgical hub 20006 may interact with one or more sensing systems 20011, one or more smart devices, and multiple displays. The surgical hub 20006 may be configured to gather measurement data from the sensing system(s) and send notifications or control messages to the one or more sensing systems 20011. The surgical hub 20006 may send and/or receive information including notification information to and/or from the human interface system 20012. The human interface system 20012 may include one or more human interface devices (HIDs). The surgical hub 20006 may send and/or receive notification information or control information to audio, display and/or control information to various devices that are in communication with the surgical hub.

For example, the sensing systems may include the wearable sensing system 20011 (which may include one or more HCP sensing systems and/or one or more patient sensing systems) and/or the environmental sensing system 20015 shown in FIG. 1. The sensing system(s) may measure data relating to various biomarkers. The sensing system(s) may measure the biomarkers using one or more sensors, for example, photosensors (e.g., photodiodes, photoresistors), mechanical sensors (e.g., motion sensors), acoustic sensors, electrical sensors, electrochemical sensors, thermoelectric sensors, infrared sensors, etc. The sensor(s) may measure the biomarkers as described herein using one of more of the following sensing technologies: photoplethysmography, electrocardiography, electroencephalography, colorimetry, impedimentary, potentiometry, amperometry, etc.

The biomarkers measured by the sensing systems may include, but are not limited to, sleep, core body temperature, maximal oxygen consumption, physical activity, alcohol consumption, respiration rate, oxygen saturation, blood pressure, blood sugar, heart rate variability, blood potential of hydrogen, hydration state, heart rate, skin conductance, peripheral temperature, tissue perfusion pressure, coughing and sneezing, gastrointestinal motility, gastrointestinal tract imaging, respiratory tract bacteria, edema, mental aspects, sweat, circulating tumor cells, autonomic tone, circadian rhythm, and/or menstrual cycle.

The biomarkers may relate to physiologic systems, which may include, but are not limited to, behavior and psychology, cardiovascular system, renal system, skin system, nervous system, gastrointestinal system, respiratory system, endocrine system, immune system, tumor, musculoskeletal system, and/or reproductive system. Information from the biomarkers may be determined and/or used by the computer-implemented patient and the surgical system 20000, for example. The information from the biomarkers may be determined and/or used by the computer-implemented patient and the surgical system 20000 to improve said systems and/or to improve patient outcomes, for example.

The sensing systems may send data to the surgical hub 20006. The sensing systems may use one or more of the following RF protocols for communicating with the surgical hub 20006: Bluetooth, Bluetooth Low-Energy (BLE), Bluetooth Smart, Zigbee, Z-wave, IPv6 Low-power wireless Personal Area Network (6LoWPAN), Wi-Fi.

The sensing systems, biomarkers, and physiological systems are described in more detail in U.S. App No. 17/156,287 (attorney docket number END9290USNP1), titled METHOD OF ADJUSTING A SURGICAL PARAMETER BASED ON BIOMARKER MEASUREMENTS, filed January 22, 2021, the disclosure of which is herein incorporated by reference in its entirety.

The sensing systems described herein may be employed to assess physiological conditions of a surgeon operating on a patient or a patient being prepared for a surgical procedure or a patient recovering after a surgical procedure. The cloud-based computing system 20008 may be used to monitor biomarkers associated with a surgeon or a patient in real-time and to generate surgical plans based at least on measurement data gathered prior to a surgical procedure, provide control signals to the surgical instruments during a surgical procedure, and notify a patient of a complication during post-surgical period.

The cloud-based computing system 20008 may be used to analyze surgical data. Surgical data may be obtained via one or more intelligent instrument(s) 20014, wearable sensing system(s) 20011, environmental sensing system(s) 20015, robotic system(s) 20013 and/or the like in the surgical system 20002. Surgical data may include tissue states to assess leaks or perfusion of sealed tissue after a tissue sealing and cutting procedure pathology data, including images of samples of body tissue, anatomical structures of the body using a variety of sensors integrated with imaging devices and techniques such as overlaying images captured by multiple imaging devices, image data, and/or the like. The surgical data may be analyzed to improve surgical procedure outcomes by determining if further treatment, such as the application of endoscopic intervention, emerging technologies, a targeted radiation, targeted intervention, and precise robotics to tissue-specific sites and conditions. Such data analysis may employ outcome analytics processing and using standardized approaches may provide beneficial feedback to either confirm surgical treatments and the behavior of the surgeon or suggest modifications to surgical treatments and the behavior of the surgeon.

FIG. 2 shows an example surgical system 20002 in a surgical operating room. As illustrated in FIG. 2, a patient is being operated on by one or more health care professionals (HCPs). The HCPs are being monitored by one or more HCP sensing systems 20020 worn by the HCPs. The HCPs and the environment surrounding the HCPs may also be monitored by one or more environmental sensing systems including, for example, a set of cameras 20021, a set of microphones 20022, and other sensors that may be deployed in the operating room. The HCP sensing systems 20020 and the environmental sensing systems may be in communication with a surgical hub 20006, which in turn may be in communication with one or more cloud servers 20009 of the cloud computing system 20008, as shown in FIG. 1. The environmental sensing systems may be used for measuring one or more environmental attributes, for example, HCP position in the surgical theater, HCP movements, ambient noise in the surgical theater, temperature/humidity in the surgical theater, etc.

As illustrated in FIG. 2, a primary display 20023 and one or more audio output devices (e.g., speakers 20019) are positioned in the sterile field to be visible to an operator at the operating table 20024. In addition, a visualization/notification tower 20026 is positioned outside the sterile field. The visualization/notification tower 20026 may include a first non-sterile human interactive device (HID) 20027 and a second non-sterile HID 20029, which may face away from each other. The HID may be a display or a display with a touchscreen allowing a human to interface directly with the HID. A human interface system, guided by the surgical hub 20006, may be configured to utilize the HIDs 20027, 20029, and 20023 to coordinate information flow to operators inside and outside the sterile field. In an example, the surgical hub 20006 may cause an HID (e.g., the primary HID 20023) to display a notification and/or information about the patient and/or a surgical procedure step. In an example, the surgical hub 20006 may prompt for and/or receive input from personnel in the sterile field or in the non-sterile area. In an example, the surgical hub 20006 may cause an HID to display a snapshot of a surgical site, as recorded by an imaging device 20030, on a non-sterile HID 20027 or 20029, while maintaining a live feed of the surgical site on the primary HID 20023. The snapshot on the non-sterile display 20027 or 20029 can permit a non-sterile operator to perform a diagnostic step relevant to the surgical procedure, for example.

The surgical hub 20006 may be configured to route a diagnostic input or feedback entered by a non-sterile operator at the visualization tower 20026 to the primary display 20023 within the sterile field, where it can be viewed by a sterile operator at the operating table. In an example, the input can be in the form of a modification to the snapshot displayed on the non-sterile display 20027 or 20029, which can be routed to the primary display 20023 by the surgical hub 20006.

Referring to FIG. 2, a surgical instrument 20031 is being used in the surgical procedure as part of the surgical system 20002. The hub 20006 may be configured to coordinate information flow to a display of the surgical instrument(s) 20031. For example, in U.S. Patent Application Publication No. US 2019-0200844 A1 (U.S. Patent Application No. 16/209,385), titled METHOD OF HUB COMMUNICATION, PROCESSING, STORAGE AND DISPLAY, filed December 4, 2018, the disclosure of which is herein incorporated by reference in its entirety. A diagnostic input or feedback entered by a non-sterile operator at the visualization tower 20026 can be routed by the hub 20006 to the surgical instrument display within the sterile field, where it can be viewed by the operator of the surgical instrument 20031. Example surgical instruments that are suitable for use with the surgical system 20002 are described under the heading "Surgical Instrument Hardware" and in U.S. Patent Application Publication No. US 2019-0200844 A1 (U.S. Patent Application No. 16/209,385), titled METHOD OF HUB COMMUNICATION, PROCESSING, STORAGE AND DISPLAY, filed December 4, 2018, the disclosure of which is herein incorporated by reference in its entirety, for example.

As shown in FIG. 2, the surgical system 20002 can be used to perform a surgical procedure on a patient who is lying down on an operating table 20024 in a surgical operating room 20035. A robotic system 20034 may be used in the surgical procedure as a part of the surgical system 20002. The robotic system 20034 may include a surgeon's console 20036, a patient side cart 20032 (surgical robot), and a surgical robotic hub 20033. The patient side cart 20032 can manipulate at least one removably coupled surgical tool 20037 through a minimally invasive incision in the body of the patient while the surgeon views the surgical site through the surgeon's console 20036. An image of the surgical site can be obtained by a medical imaging device 20030, which can be manipulated by the patient side cart 20032 to orient the imaging device 20030. The robotic hub 20033 can be used to process the images of the surgical site for subsequent display to the surgeon through the surgeon's console 20036.

Other types of robotic systems can be readily adapted for use with the surgical system 20002. Various examples of robotic systems and surgical tools that are suitable for use with the present disclosure are described herein, as well as in U.S. Patent Application Publication No. US 2019-0201137 A1 (U.S. Patent Application No. 16/209,407), titled METHOD OF ROBOTIC HUB COMMUNICATION, DETECTION, AND CONTROL, filed December 4, 2018, the disclosure of which is herein incorporated by reference in its entirety.

In various aspects, the imaging device 20030 may include at least one image sensor and one or more optical components. Suitable image sensors may include, but are not limited to, Charge-Coupled Device (CCD) sensors and Complementary Metal-Oxide Semiconductor (CMOS) sensors.

The optical components of the imaging device 20030 may include one or more illumination sources and/or one or more lenses. The one or more illumination sources may be directed to illuminate portions of the surgical field. The one or more image sensors may receive light reflected or refracted from the surgical field, including light reflected or refracted from tissue and/or surgical instruments.

The illumination source(s) may be configured to radiate electromagnetic energy in the visible spectrum as well as the invisible spectrum. The visible spectrum, sometimes referred to as the optical spectrum or luminous spectrum, is the portion of the electromagnetic spectrum that is visible to (e.g., can be detected by) the human eye and may be referred to as visible light or simply light. A typical human eye will respond to wavelengths in air that range from about 380 nm to about 750 nm.

The invisible spectrum (e.g., the non-luminous spectrum) is the portion of the electromagnetic spectrum that lies below and above the visible spectrum (i.e., wavelengths below about 380 nm and above about 750 nm). The invisible spectrum is not detectable by the human eye. Wavelengths greater than about 750 nm are longer than the red visible spectrum, and they become invisible infrared (IR), microwave, and radio electromagnetic radiation. Wavelengths less than about 380 nm are shorter than the violet spectrum, and they become invisible ultraviolet, x-ray, and gamma ray electromagnetic radiation.

In various aspects, the imaging device 20030 is configured for use in a minimally invasive procedure. Examples of imaging devices suitable for use with the present disclosure include, but are not limited to, an arthroscope, angioscope, bronchoscope, choledochoscope, colonoscope, cytoscope, duodenoscope, enteroscope, esophagogastro-duodenoscope (gastroscope), endoscope, laryngoscope, nasopharyngo-neproscope, sigmoidoscope, thoracoscope, and ureteroscope.

The imaging device may employ multi-spectrum monitoring to discriminate topography and underlying structures. A multi-spectral image is one that captures image data within specific wavelength ranges across the electromagnetic spectrum. The wavelengths may be separated by filters or by the use of instruments that are sensitive to particular wavelengths, including light from frequencies beyond the visible light range, e.g., IR and ultraviolet. Spectral imaging can allow extraction of additional information that the human eye fails to capture with its receptors for red, green, and blue. The use of multi-spectral imaging is described in greater detail under the heading "Advanced Imaging Acquisition Module" in U.S. Patent Application Publication No. US 2019-0200844 A1 (U.S. Patent Application No. 16/209,385), titled METHOD OF HUB COMMUNICATION, PROCESSING, STORAGE AND DISPLAY, filed December 4, 2018, the disclosure of which is herein incorporated by reference in its entirety. Multi-spectrum monitoring can be a useful tool in relocating a surgical field after a surgical task is completed to perform one or more of the previously described tests on the treated tissue. It is axiomatic that strict sterilization of the operating room and surgical equipment is required during any surgery. The strict hygiene and sterilization conditions required in a "surgical theater," e.g., an operating or treatment room, necessitate the highest possible sterility of all medical devices and equipment. Part of that sterilization process is the need to sterilize anything that comes in contact with the patient or penetrates the sterile field, including the imaging device 20030 and its attachments and components. It will be appreciated that the sterile field may be considered a specified area, such as within a tray or on a sterile towel, that is considered free of microorganisms, or the sterile field may be considered an area, immediately around a patient, who has been prepared for a surgical procedure. The sterile field may include the scrubbed team members, who are properly attired, and all furniture and fixtures in the area.

Wearable sensing system 20011 illustrated in FIG. 1 may include one or more HCP sensing systems 20020 as shown in FIG. 2. The HCP sensing systems 20020 may include sensing systems to monitor and detect a set of physical states and/or a set of physiological states of a healthcare personnel (HCP). An HCP may be a surgeon or one or more healthcare personnel assisting the surgeon or other healthcare service providers in general. In an example, an HCP sensing system 20020 may measure a set of biomarkers to monitor the heart rate of an HCP. In an example, an HCP sensing system 20020 worn on a surgeon's wrist (e.g., a watch or a wristband) may use an accelerometer to detect hand motion and/or shakes and determine the magnitude and frequency of tremors. The sensing system 20020 may send the measurement data associated with the set of biomarkers and the data associated with a physical state of the surgeon to the surgical hub 20006 for further processing.

The environmental sensing system(s) 20015 shown in FIG. 1 may send environmental information to the surgical hub 20006. For example, the environmental sensing system(s) 20015 may include a camera 20021 for detecting hand/body position of an HCP. The environmental sensing system(s) 20015 may include microphones 20022 for measuring the ambient noise in the surgical theater. Other environmental sensing system(s) 20015 may include devices, for example, a thermometer to measure temperature and a hygrometer to measure humidity of the surroundings in the surgical theater, etc. The surgeon biomarkers may include one or more of the following: stress, heart rate, etc. The environmental measurements from the surgical theater may include ambient noise level associated with the surgeon or the patient, surgeon and/or staff movements, surgeon and/or staff attention level, etc. The surgical hub 20006, alone or in communication with the cloud computing system, may use the surgeon biomarker measurement data and/or environmental sensing information to modify the control algorithms of hand-held instruments or the averaging delay of a robotic interface, for example, to minimize tremors.

The surgical hub 20006 may use the surgeon biomarker measurement data associated with an HCP to adaptively control one or more surgical instruments 20031. For example, the surgical hub 20006 may send a control program to a surgical instrument 20031 to control its actuators to limit or compensate for fatigue and use of fine motor skills. The surgical hub 20006 may send the control program based on situational awareness and/or the context on importance or criticality of a task. The control program may instruct the instrument to alter operation to provide more control when control is needed.

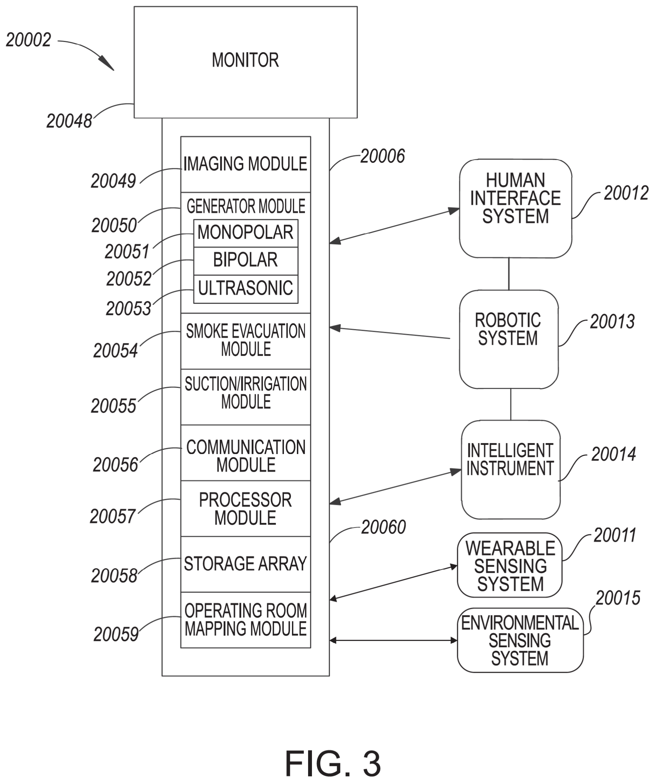

FIG. 3 shows an example surgical system 20002 with a surgical hub 20006. The surgical hub 20006 may be paired with, via a modular control, a wearable sensing system 20011, an environmental sensing system 20015, a human interface system 20012, a robotic system 20013, and an intelligent instrument 20014. The hub 20006 includes a display 20048, an imaging module 20049, a generator module 20050 (e.g., an energy generator), a communication module 20056, a processor module 20057, a storage array 20058, and an operating-room mapping module 20059. In certain aspects, as illustrated in FIG. 3, the hub 20006 further includes a smoke evacuation module 20054 and/or a suction/irrigation module 20055. The various modules and systems may be connected to the modular control either directly via a router or via the communication module 20056. The operating theater devices may be coupled to cloud computing resources and data storage via the modular control. The human interface system 20012 may include a display sub-system and a notification sub-system.

The modular control may be coupled to non-contact sensor module. The non-contact sensor module may measure the dimensions of the operating theater and generate a map of the surgical theater using ultrasonic, laser-type, and/or the like, non-contact measurement devices. Other distance sensors can be employed to determine the bounds of an operating room. An ultrasound-based non-contact sensor module may scan the operating theater by transmitting a burst of ultrasound and receiving the echo when it bounces off the perimeter walls of an operating theater as described under the heading "Surgical Hub Spatial Awareness Within an Operating Room" in U.S. Provisional Patent Application Serial No. 62/611,341, titled INTERACTIVE SURGICAL PLATFORM, filed December 28, 2017, which is herein incorporated by reference in its entirety. The sensor module may be configured to determine the size of the operating theater and to adjust Bluetooth-pairing distance limits. A laser-based non-contact sensor module may scan the operating theater by transmitting laser light pulses, receiving laser light pulses that bounce off the perimeter walls of the operating theater, and comparing the phase of the transmitted pulse to the received pulse to determine the size of the operating theater and to adjust Bluetooth pairing distance limits, for example.

During a surgical procedure, energy application to tissue, for sealing and/or cutting, may be associated with smoke evacuation, suction of excess fluid, and/or irrigation of the tissue. Fluid, power, and/or data lines from different sources may be entangled during the surgical procedure. Valuable time can be lost addressing this issue during a surgical procedure. Detangling the lines may necessitate disconnecting the lines from their respective modules, which may require resetting the modules. The hub modular enclosure 20060 may offer a unified environment for managing the power, data, and fluid lines, which reduces the frequency of entanglement between such lines.

Energy may be applied to tissue at a surgical site. The surgical hub 20006 may include a hub enclosure 20060 and a combo generator module slidably receivable in a docking station of the hub enclosure 20060. The docking station may include data and power contacts. The combo generator module may include two or more of: an ultrasonic energy generator component, a bipolar RF energy generator component, or a monopolar RF energy generator component that are housed in a single unit. The combo generator module may include a smoke evacuation component, at least one energy delivery cable for connecting the combo generator module to a surgical instrument, at least one smoke evacuation component configured to evacuate smoke, fluid, and/or particulates generated by the application of therapeutic energy to the tissue, and a fluid line extending from the remote surgical site to the smoke evacuation component. The fluid line may be a first fluid line, and a second fluid line may extend from the remote surgical site to a suction and irrigation module 20055 slidably received in the hub enclosure 20060. The hub enclosure 20060 may include a fluid interface.

The combo generator module may generate multiple energy types for application to the tissue. One energy type may be more beneficial for cutting the tissue, while another different energy type may be more beneficial for sealing the tissue. For example, a bipolar generator can be used to seal the tissue while an ultrasonic generator can be used to cut the sealed tissue. Aspects of the present disclosure present a solution where a hub modular enclosure 20060 is configured to accommodate different generators and facilitate an interactive communication therebetween. The hub modular enclosure 20060 may enable the quick removal and/or replacement of various modules.

The modular surgical enclosure may include a first energy-generator module, configured to generate a first energy for application to the tissue, and a first docking station comprising a first docking port that includes first data and power contacts, wherein the first energy-generator module is slidably movable into an electrical engagement with the power and data contacts and wherein the first energy-generator module is slidably movable out of the electrical engagement with the first power and data contacts. The modular surgical enclosure may include a second energy-generator module configured to generate a second energy, different than the first energy, for application to the tissue, and a second docking station comprising a second docking port that includes second data and power contacts, wherein the second energy generator module is slidably movable into an electrical engagement with the power and data contacts, and wherein the second energy-generator module is slidably movable out of the electrical engagement with the second power and data contacts. In addition, the modular surgical enclosure also includes a communication bus between the first docking port and the second docking port, configured to facilitate communication between the first energy-generator module and the second energy-generator module.

Referring to FIG. 3, the hub modular enclosure 20060 may allow the modular integration of a generator module 20050, a smoke evacuation module 20054, and a suction/irrigation module 20055. The hub modular enclosure 20060 may facilitate interactive communication between the modules 20059, 20054, and 20055. The generator module 20050 can be with integrated monopolar, bipolar, and ultrasonic components supported in a single housing unit slidably insertable into the hub modular enclosure 20060. The generator module 20050 may connect to a monopolar device 20051, a bipolar device 20052, and an ultrasonic device 20053. The generator module 20050 may include a series of monopolar, bipolar, and/or ultrasonic generator modules that interact through the hub modular enclosure 20060. The hub modular enclosure 20060 may facilitate the insertion of multiple generators and interactive communication between the generators docked into the hub modular enclosure 20060 so that the generators would act as a single generator.

A surgical data network having a set of communication hubs may connect the sensing system(s), the modular devices located in one or more operating theaters of a healthcare facility, a patient recovery room, or a room in a healthcare facility specially equipped for surgical operations, to the cloud computing system 20008.

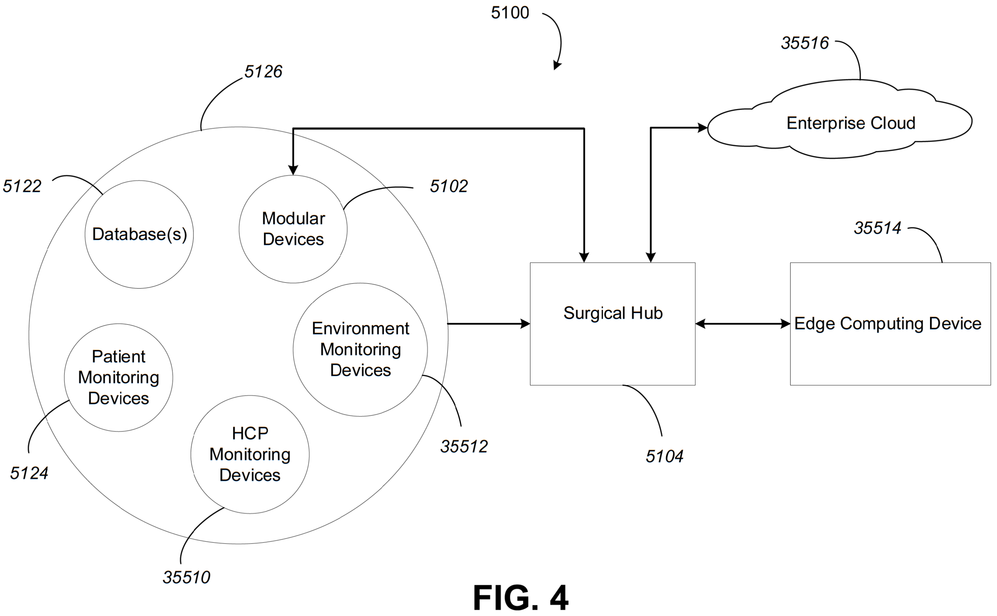

FIG. 4 illustrates a diagram of a situationally aware surgical system 5100. The data sources 5126 may include, for example, the modular devices 5102, databases 5122 (e.g., an EMR database containing patient records), patient monitoring devices 5124 (e.g., a blood pressure (BP) monitor and an electrocardiography (EKG) monitor), HCP monitoring devices 35510, and/or environment monitoring devices 35512. The modular devices 5102 may include sensors configured to detect parameters associated with the patient, HCPs and environment and/or the modular device itself. The modular devices 5102 may include one or more intelligent instrument(s) 20014. The surgical hub 5104 may derive the contextual information pertaining to the surgical procedure from the data based upon, for example, the particular combination(s) of received data or the particular order in which the data is received from the data sources 5126. The contextual information inferred from the received data can include, for example, the type of surgical procedure being performed, the particular step of the surgical procedure that the surgeon is performing, the type of tissue being operated on, or the body cavity that is the subject of the procedure. This ability by some aspects of the surgical hub 5104 to derive or infer information related to the surgical procedure from received data can be referred to as "situational awareness." For example, the surgical hub 5104 can incorporate a situational awareness system, which may be the hardware and/or programming associated with the surgical hub 5104 that derives contextual information pertaining to the surgical procedure from the received data and/or a surgical plan information received from the edge computing system 35514 or an enterprise cloud server 35516. The contextual information derived from the data sources 5126 may include, for example, what step of the surgical procedure is being performed, whether and how a particular modular device 5102 is being used, and the patient's condition.

The surgical hub 5104 may be connected to various databases 5122 to retrieve therefrom data regarding the surgical procedure that is being performed or is to be performed. In one exemplification of the surgical system 5100, the databases 5122 may include an EMR database of a hospital. The data that may be received by the situational awareness system of the surgical hub 5104 from the databases 5122 may include, for example, start (or setup) time or operational information regarding the procedure (e.g., a segmentectomy in the upper right portion of the thoracic cavity). The surgical hub 5104 may derive contextual information regarding the surgical procedure from this data alone or from the combination of this data and data from other data sources 5126.

The surgical hub 5104 may be connected to (e.g., paired with) a variety of patient monitoring devices 5124. In an example of the surgical system 5100, the patient monitoring devices 5124 that can be paired with the surgical hub 5104 may include a pulse oximeter (SpO2 monitor) 5114, a BP monitor 5116, and an EKG monitor 5120. The perioperative data that is received by the situational awareness system of the surgical hub 5104 from the patient monitoring devices 5124 may include, for example, the patient's oxygen saturation, blood pressure, heart rate, and other physiological parameters. The contextual information that may be derived by the surgical hub 5104 from the perioperative data transmitted by the patient moni-toring devices 5124 may include, for example, whether the patient is located in the operating theater or under anesthesia. The surgical hub 5104 may derive these inferences from data from the patient monitoring devices 5124 alone or in combination with data from other data sources 5126 (e.g., the ventilator 5118).

The surgical hub 5104 may be connected to (e.g., paired with) a variety of modular devices 5102. In one exemplification of the surgical system 5100, the modular devices 5102 that are paired with the surgical hub 5104 may include a smoke evacuator, a medical imaging device such as the imaging device 20030 shown in FIG. 2, an insufflator, a combined energy generator (for powering an ultrasonic surgical instrument and/or an RF electrosurgical instrument), and a ventilator.

The perioperative data received by the surgical hub 5104 from the medical imaging device may include, for example, whether the medical imaging device is activated and a video or image feed. The contextual information that is derived by the surgical hub 5104 from the perioperative data sent by the medical imaging device may include, for example, whether the procedure is a VATS procedure (based on whether the medical imaging device is activated or paired to the surgical hub 5104 at the beginning or during the course of the procedure). The image or video data from the medical imaging device (or the data stream representing the video for a digital medical imaging device) may be processed by a pattern recognition system or a machine learning system to recognize features (e.g., organs or tissue types) in the field of view (FOY) of the medical imaging device, for example. The contextual information that is derived by the surgical hub 5104 from the recognized features may include, for example, what type of surgical procedure (or step thereof) is being performed, what organ is being operated on, or what body cavity is being operated in.

The situational awareness system of the surgical hub 5104 may derive the contextual information from the data received from the data sources 5126 in a variety of different ways. For example, the situational awareness system can include a pattern recognition system, or machine learning system (e.g., an artificial neural network), that has been trained on training data to correlate various inputs (e.g., data from database(s) 5122, patient monitoring devices 5124, modular devices 5102, HCP monitoring devices 35510, and/or environment monitoring devices 35512) to corresponding contextual information regarding a surgical procedure. For example, a machine learning system may accurately derive contextual information regarding a surgical procedure from the provided inputs. In examples, the situational awareness system can include a lookup table storing pre-characterized contextual information regarding a surgical procedure in association with one or more inputs (or ranges of inputs) corresponding to the contextual information. In response to a query with one or more inputs, the lookup table can return the corresponding contextual information for the situational awareness system for controlling the modular devices 5102. In examples, the contextual information received by the situational awareness system of the surgical hub 5104 can be associated with a particular control adjustment or set of control adjustments for one or more modular devices 5102. In examples, the situational awareness system can include a machine learning system, lookup table, or other such system, which may generate or retrieve one or more control adjustments for one or more modular devices 5102 when provided the contextual information as input.

For example, based on the data sources 5126, the situationally aware surgical hub 5104 may determine what type of tissue was being operated on. The situationally aware surgical hub 5104 can infer whether a surgical procedure being performed is a thoracic or an abdominal procedure, allowing the surgical hub 5104 to determine whether the tissue clamped by an end effector of the surgical stapling and cutting instrument is lung (for a thoracic procedure) or stomach (for an abdominal procedure) tissue. The situationally aware surgical hub 5104 may determine whether the surgical site is under pressure (by determining that the surgical procedure is utilizing insufflation) and determine the procedure type, for a consistent amount of smoke evacuation for both thoracic and abdominal procedures. Based on the data sources 5126, the situationally aware surgical hub 5104 could determine what step of the surgical procedure is being performed or will subsequently be performed.

The situationally aware surgical hub 5104 could determine what type of surgical procedure is being performed and customize the energy level according to the expected tissue profile for the surgical procedure. The situationally aware surgical hub 5104 may adjust the energy level for the ultrasonic surgical instrument or RF electrosurgical instrument throughout the course of a surgical procedure, rather than just on a procedure-by-procedure basis.

In examples, data can be drawn from additional data sources 5126 to improve the conclusions that the surgical hub 5104 draws from one data source 5126. The situationally aware surgical hub 5104 could augment data that it receives from the modular devices 5102 with contextual information that it has built up regarding the surgical procedure from other data sources 5126.

The situational awareness system of the surgical hub 5104 can consider the physiological measurement data to provide additional context in analyzing the visualization data. The additional context can be useful when the visualization data may be inconclusive or incomplete on its own.

The situationally aware surgical hub 5104 could determine whether the surgeon (or other HCP(s)) was making an error or otherwise deviating from the expected course of action during the course of a surgical procedure. For example, the surgical hub 5104 may determine the type of surgical procedure being performed, retrieve the corresponding list of steps or order of equipment usage (e.g., from a memory), and compare the steps being performed or the equipment being used during the course of the surgical procedure to the expected steps or equipment for the type of surgical procedure that the surgical hub 5104 determined is being performed. The surgical hub 5104 can provide an alert indicating that an unexpected action is being performed or an unexpected device is being utilized at the particular step in the surgical procedure.

The surgical instruments (and other modular devices 5102) may be adjusted for the particular context of each surgical procedure (such as adjusting to different tissue types) and validating actions during a surgical procedure. Next steps, data, and display adjustments may be provided to surgical instruments (and other modular devices 5102) in the surgical theater according to the specific context of the procedure.

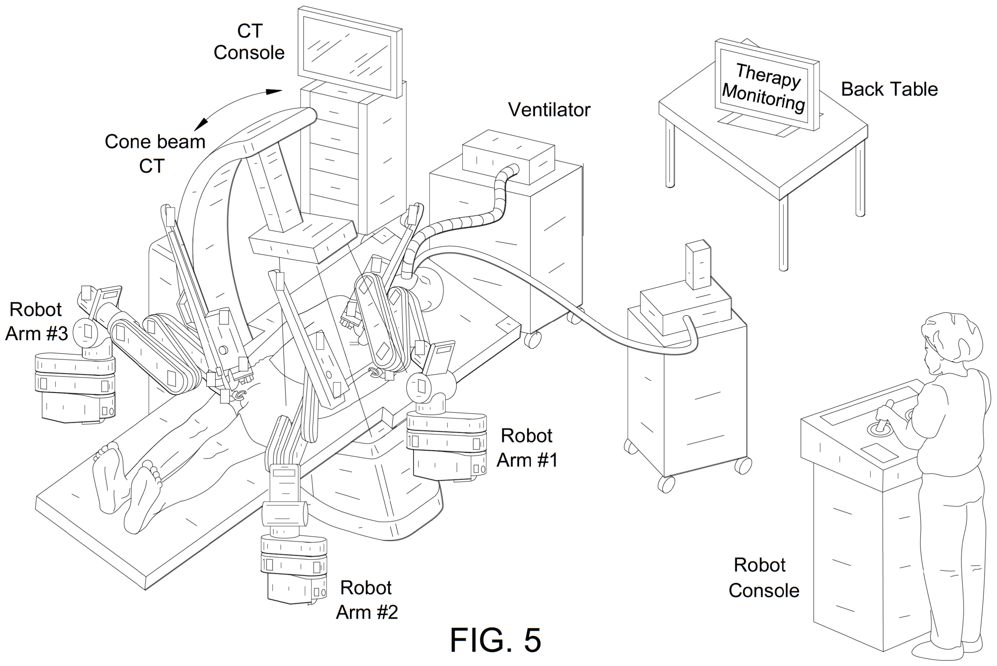

FIG. 5 illustrates an example operating room arrangement of multiple robotic arms and surgical equipment. As shown, multiple robotic arms may be present in a relatively small space in an OR. The arms may have ranges of motion that overlap with each other or other devices. In this case, the arms may collide unless adjustments are made to reduce the potential interactions. For example, one or more of the arms may not be allowed to occupy the overlapping space (e.g., at a given time).

As shown, the operating room may include one or more fixed (e.g., non-moving) devices. For example, the fixed devices may include a ventilator (e.g., a Monarch smart ventilator). The ventilator may be placed (e.g., and fixed) at the patient's head (e.g., because the ventilator must be attached at the patient's mouth and/or nose).

A smart system may display and highlight confounding data to improve feedback provided to a health care provider (HCP). The system may display complex and/or conflicting interrelated data streams to the HCP for input. Multiple monitored patient data streams (e.g., that are related to the same control parameter of a smart system and closed loop on at least one of the biomarkers) may provide inconsistent information about the control patient parameter. More than one of the parameters may be displayed to the user with context (e.g., to enable the HCP to intervene or provide guidance regarding system actions relative to the inconsistency). The related biomarkers may be from multiple smart systems and/or measured in multiple patient locations.

The interrelationship of monitored signals may be (e.g., may appear to be) conflicting or confounded. In this case, the system may not act on the signals without input from the HCP. The system may display and/or highlight combined datasets for the HCP to review. For example, the system may provide the signals and context to the HCP (e.g., so that the HCP may intervene in the decision-making process, if necessary).

An individual data stream may not function as expected. In this case, the system may be unable to proceed with an automated decision. The system may seek input from the surgeon. The system may determine data to display when asking for surgeon input (e.g., requesting that the surgeon confirm if a data stream may be not behaving as expected).

The user (e.g., surgeon) may validate one or more data streams. Data stream(s) may have undefined functionality. The system may have an undefined reaction to the introduction of the data stream(s). Data streams may be configured (e.g., in real time) to allow the user to incorporate new data streams (e.g., and validate the integrity of the data).

The HCP may generate limit(s) on the information displayed. For example, the system may display extended/long term/historic data stream, data relative to predefined limits, etc.

During surgery, the system may monitor patient temperature. If the mean body temperature while under anesthesia drops below 35°C, it may result in vasoconstriction. If the mean body temperature exceeds 37.5°C, it may result in vasodilation. The system may show the temperature of the patient relative to those limits.

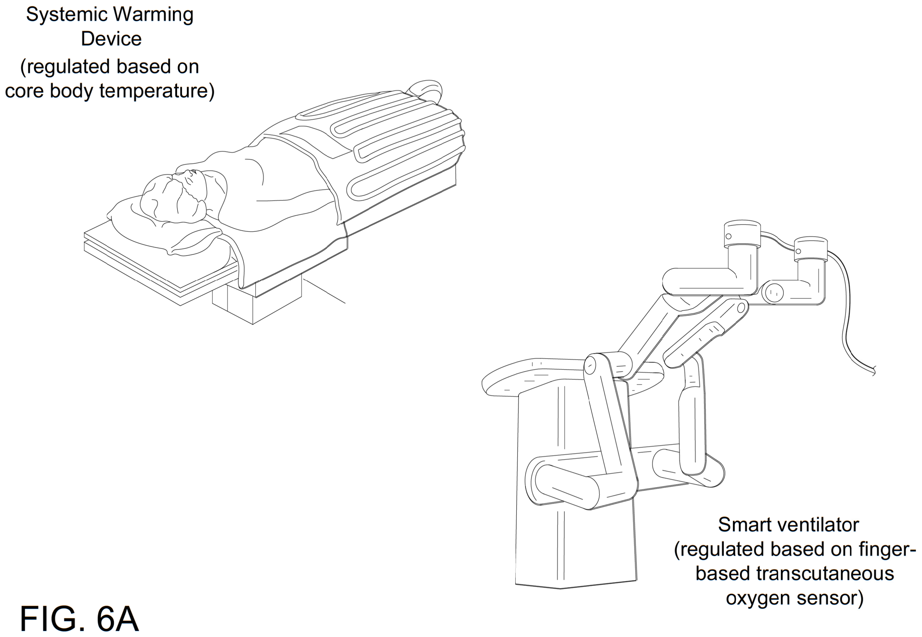

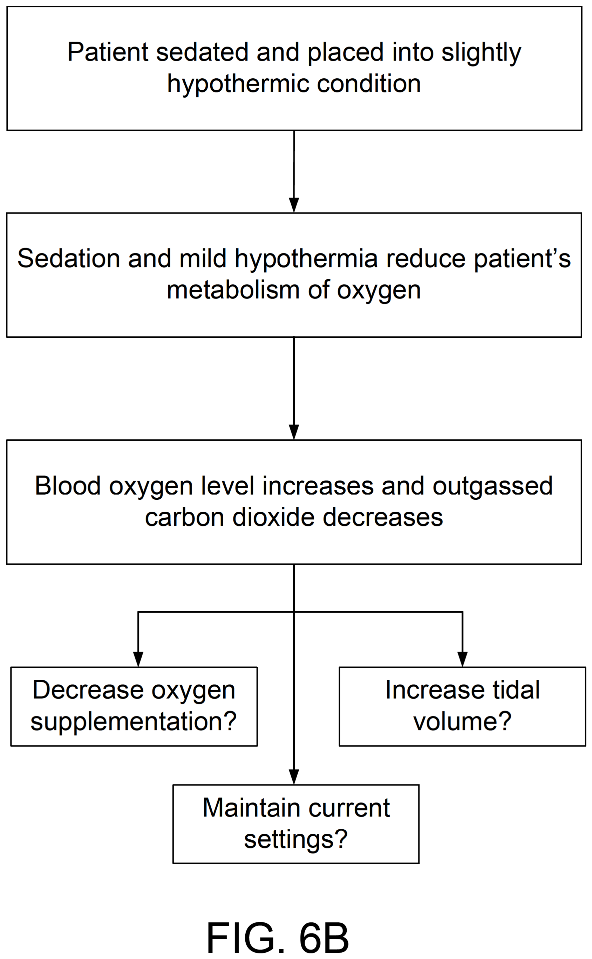

FIGs. 6A-C illustrate example conflicting decisions based on data from a systemic warming device and data from a smart ventilator. As shown in FIG. 6A, the systemic warming device may be regulated based on the core body temperature of the patient. The smart ventilator may be regulated based on finger-based sensor for monitoring transcutaneous oxygen levels. As shown in FIG. 6B, a surgical procedure may involve sedating the patient and placed in a slightly hypothermic state/condition. The sedation and mild hypothermia may reduce the patient's metabolism of oxygen. During the procedure, the blood oxygen level may increase and the outgassed carbon dioxide may decrease. In this case, the system may not know whether to decrease oxygen supplementation or increase tidal volume.

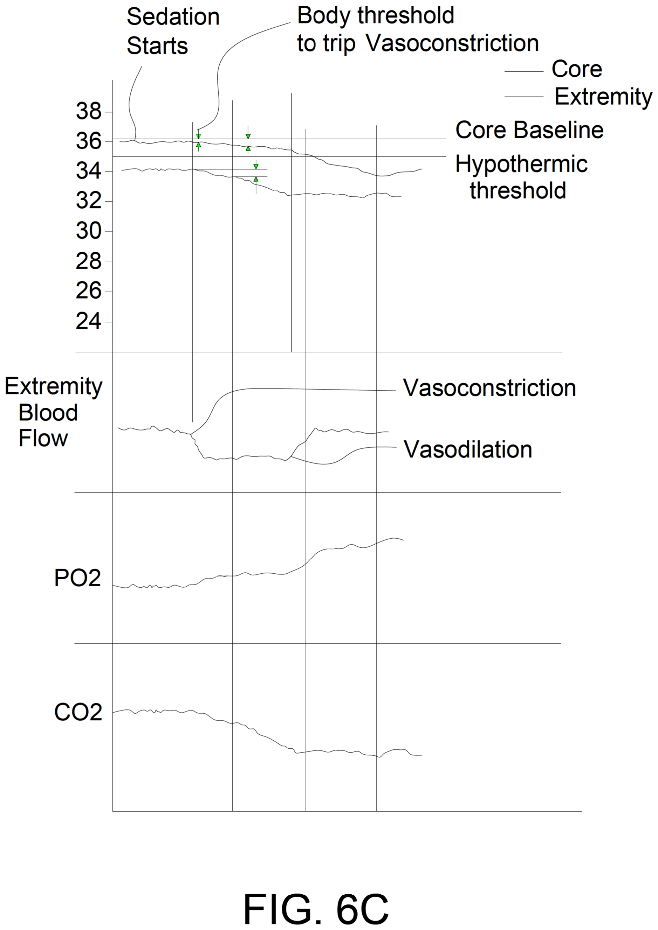

The system's decision may vary based on which temperature reading the system uses. For example, the system may select one option over the other based on the core body temperature reading but may select the other option based on the finger-based oxygen sensor. For example, as shown in FIG. 6C, the extremity temperature may drop below the hypothermic threshold before the core body temperature. The temperature change may (e.g., initially) result in vasoconstriction (e.g., as the body tried to maintain core temperature with the aid of the systemic patient warming). Specifically, the body may vasoconstrict the blood flow to extremities.

As the procedure continues, the patient's core temperature may drop below the hypothermic threshold. The body may reverse the vasoconstriction to a vasodilation state. The vasodilation opens the flow of cold blood to the extremities, which may rapidly increase the core temperature loss. As the patient's core body temperature continues to drop, the patient's blood oxygen level (PO2) may increase and the outgassed carbon dioxide (CO2) may decrease, as shown. If vasodilation occurs and the patient's temperature at the extremities increases, the system may be unsure of whether to maintain or change the oxygen supplementation and/or tidal volume.

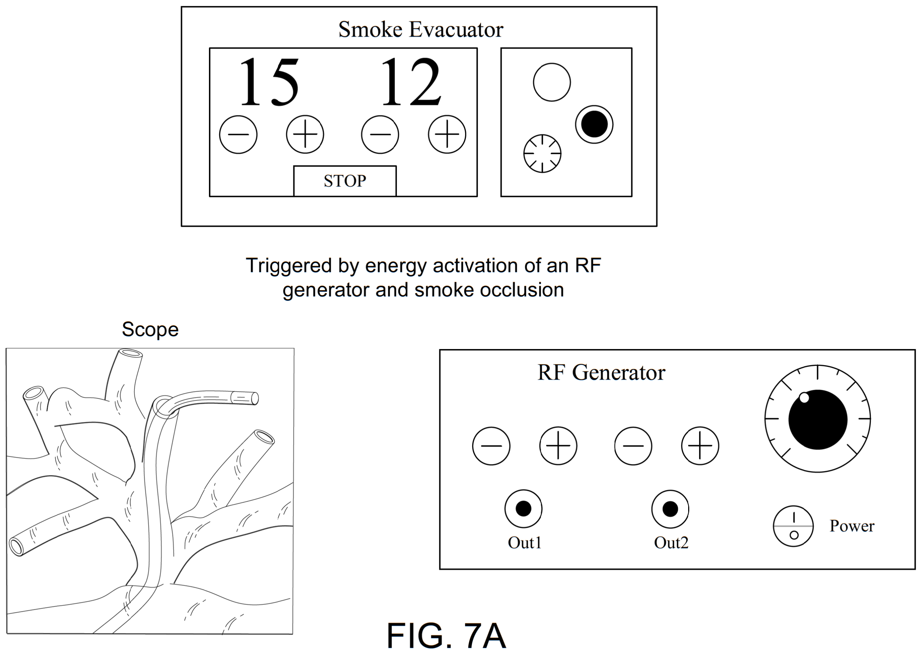

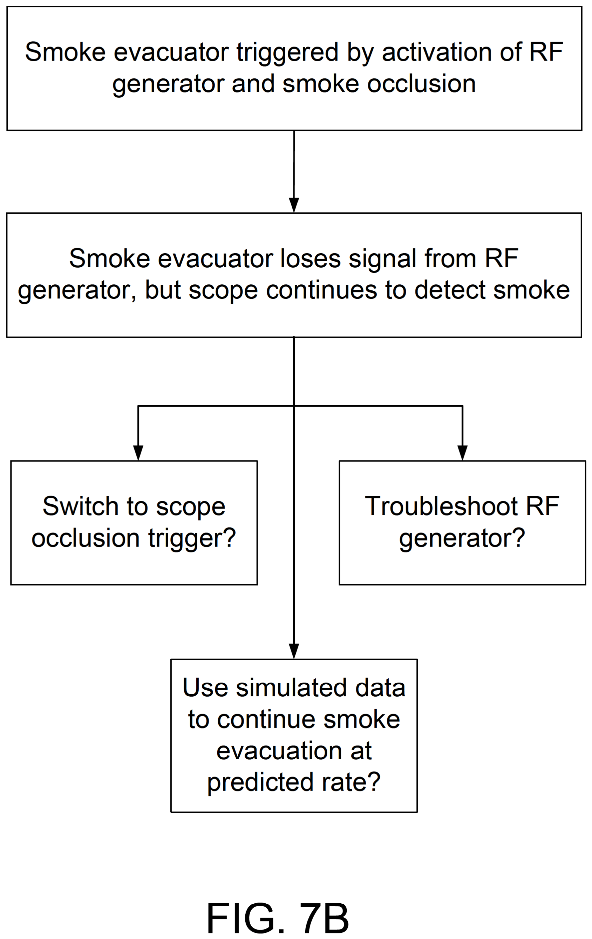

In another example, as illustrated in FIG. 7A a smoke evacuator may be activated by energy activation of an RF generator and/or smoke occlusion detection. The smoke evacuation may remove the smoke to improve visibility and reduce abdominal pressure. As illustrated in FIG. 7B, the smoke evacuator may lose signal from the RF generator (e.g., the cable between the evacuator and generator is unplugged/disconnected). The smoke evacuator may receive data from the scope that continues to indicate the presence of smoke. The smoke evacuator may not know whether to trust the data from the generator or the scope.

The system may determine that the sudden loss of the generator signal is likely due to a mechanical failure (e.g., disconnect rather than abrupt stop in energy during a surgical step). In this case, the system may determine to rely solely on occlusion as an evacuation trigger. In this case, the system may indicate the decision to the surgeon, along with context information (e.g., sudden end of energy activation unlikely at this time). The system may determine that the OR personnel should troubleshoot the generator. In this case, the system may display a warning that the generator may not be acting as expected. The system may determine to use simulated data to continue the smoke evacuation at a predicted rate. For example, if the historical data of smoke evacuation showed a steady decline over the previous 10 minutes, the system may continue to slowly decrease the smoke evacuation at the same rate.

Potentially problematic data may be displayed relative to the limits (e.g., previously established limits). The limits may be empirically set (e.g., based off of the limits of equipment, biological function, or established literature). The limits may be configurable to surgeon preferences. Such limits may help the system identify flawed data. For example, if the patient's temperature reaches over 212°C, it may be highly likely the data source itself may be in error.

The system may flag inaccurate data on a display. The inaccurate information may be flagged (e.g., with a red boundary) and displayed so that the surgeon can monitor the value to make a decision.

The system may display data that is relative to historical zones of interest. The system may subdivide the graphical space (e.g., based on limit(s) and/or other metrics). A graphical representation may have multiple zones. The zones may include limit(s) and/or additional zones that may be of interest to the surgeon.

Zoned data may be correlated to intensity of display graphs. For example, a visual representation of a standard deviation curve may have the intensity of a color correlated to the commonality of a value (e.g., a more common value has higher intensity, and outliers lose coloration).

In another example, a visual representation may overlay a sample standard deviation curve onto a graphical format. The coloration may be utilized to display intensity within the graph.

The system may display one or more versions of correlated data. The system may display predicted data. For example, data may include predicted data values. The predicted data values may be based on one or more models (e.g., human physiology, cause-effect, advanced machine-learning based models, etc.).

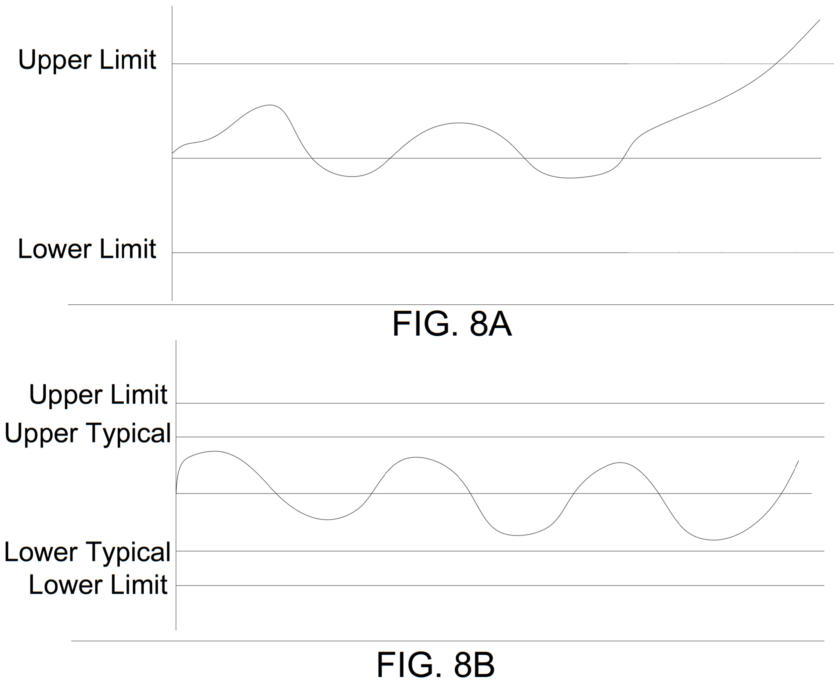

Background information may be used to provide context for decision making. The system may display data within bounds/thresholds. For example, FIG. 8A illustrates an example display of data and associated upper and lower limits. This may enable a user to see changes that don't justify a warning. If the value of the data exceeds the upper limit, as shown, the system may output a warning to the user.

The system may display conflicting data sources to the surgeon. The system may display information from multiple data sources. The user may use the display to understand how the differences may be impacting the data stream.

The system may display the current reading of a data stream. The real-time or current data may be displayed to the HCP at the same time as correlated data is displayed to the HCP.

The duration and history of data display may be configurable. Timeframes and mathematical operations (e.g., average, maximum, minimum, etc.) may be configurable by the surgeon (e.g., to best represent the information they would like to see). For example, the surgeon may request that they system display the current patient temperature and the patient average temperature over the last 10 minutes.

The system may indicate a direction and/or rate of change of a data stream. The system may indicate whether a value is increasing, decreasing, or holding steady. The system may display system behavior and/or changes. The system may indicate a transformation or compensation applied to a data stream (e.g., without showing the raw data).

The system may request user input on a data stream. For example, the system may display prompts or suggestions of how to correct the drop-out in signal. For example, the system may prompt the user for a troubleshooting step for a sensor. In an example, if the system detects that a signal dropped, the system may determine that the cable has likely been disconnected. In this case, the system may prompt the user to reconnect the cable of the system.

The system may display options (e.g., possible options moving forward) from which the user can select. The system may prompt the user with troubleshooting steps and/or actions to be taken (e.g., based on proximity and time to complete each step).

For example, the system may detect a loss in signal based on receiving corrupted or erroneous data (e.g., the voltage on the sensor may be outside the normal range). The system may prompt the user with a series of walkthroughs for how to troubleshoot the issue. For example, the troubleshooting may include checking that the cable is physically connected, checking the connection to the patient, checking that IFU steps were followed (e.g., the patient was shaved, the connection is in the correct location, etc.), replacing the sensor (e.g., if necessary), and/or the like.

The system may indicate (e.g., highlight) impacts from the data loss. The system may indicate the impact that the lost data will have on the system and/or HCP.

The system may be recalibrated using alternate data streams. Humans may monitor data streams. The system may be recalibrated to a new data stream that involves more active human monitoring.

The system may be recalibrated to a new data stream that has an additional error or offset (e.g., while remaining acceptable). In this case, the accuracy or precision that the system provides may be reduced. For example, if the primary patient temperature monitoring system fails, the system may monitor patient temperature through a finger sensor (e.g., which may not provide the same accuracy as the primary sensor). In this case, system accuracy may be reduced. The system may warn the HCP of the accuracy reduction and the change in monitoring method.

Data streams with may not impact the user or procedure. For example, if data is lost, the HCP may manually map a different data source (e.g., so that no additional action is needed).

The user may decide to proceed with the current data stream. A data stream may fall outside of a given range that was enabled but may not be physiologically incorrect. For alarms to be useful, they may be constrained to 95% of the population (e.g., because the other 5% of the population may have physiological traits that fall outside that range). In this case, the system may change a threshold/range to account for the people outside the standard range.

For example, as shown in FIG. 8B, the system may display upper and lower limits (e.g., absolute upper and lower limits), an upper typical limit, and a lower typical limit. The typical limits may show the standard limits (e.g., for 95% of the population). The absolute limits may show the absolute acceptable range of the data (e.g., for 100% of the population). For example, a heart rate monitor may determine that the patient's heart rate is very low. In this case, the system may determine whether the patient has a naturally low heart rate (e.g., below average resting heart rate). If the patient has a naturally low heart rate, the system may forego displaying a warning until the heart rate drops below to absolute lower limit.

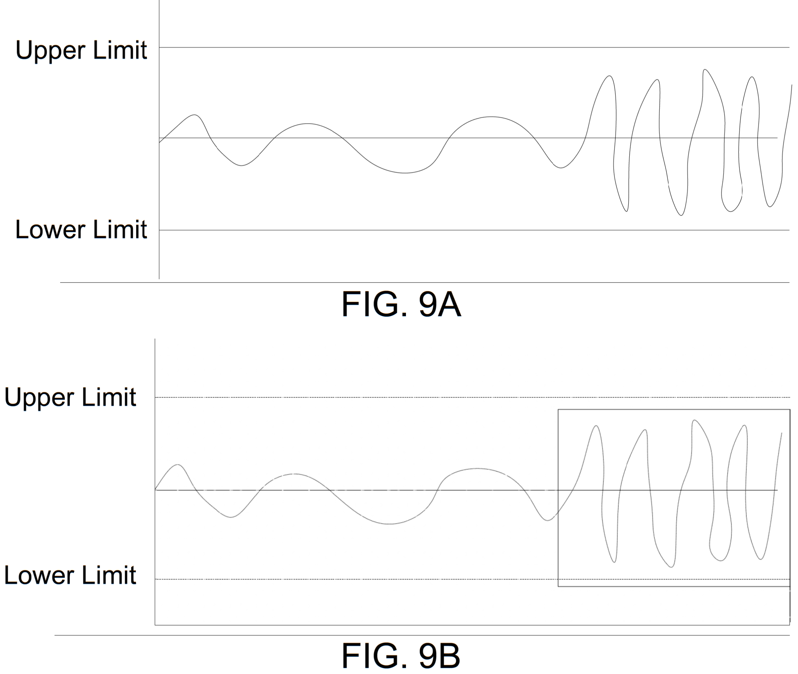

Current data and correlated data may be displayed (e.g., simultaneously). The system may display the data over time, as shown in FIG. 9A. The system may display the current value of the data and the value trending over time. The data may be displayed within a graphical format (e.g., to represent performance of the data). For example, within a monitoring system, the system may (e.g., simultaneously) display the current value of the data stream and historical data (e.g., the immediately or configured historical data) that led to the current value.

The system may display the current value in the context of (e.g., contextualized to) a correlated data stream. For example, data may be displayed for a particular reading in the context of other correlated data. Displaying a data stream may involve displaying (e.g., directly or indirectly) a plethora of (e.g., related) data. The system may utilize a line-graph, scatterplot type format, or other formats. The prior data may be related to other surgeries, or stages of those surgeries.

Data may be displayed alongside a separate (e.g., related or correlated) data stream. The system may highlight a localized data segment within a data stream. For example, if there are substantial variations in the data relative to a prior period of time, the system may display variation relative to historical trending of the data (e.g., to quickly indicate that there may be a problem present).

In an example, a baseline graphical representation may include a high variability segment. The high variability segment may be highlighted to draw attention, as shown in FIG. 9B. Segments (e.g., highlighted segments) may be detected by conventional mathematical means (e.g., measures of statistical variability, maxima or minima values, sudden changes in rate of change of a signal, machine learning, and/or the like).

The system may display historical data. Historical data may be data from a prior event. The historical data may be from a prior surgery of the same patient, performed by the same surgeon, the same hospital system, or large-scale (e.g., nationwide) surgical data. The data may include procedure-specific data, hospital-specific data, surgeon-specific data, patient-specific data, and/or the like.

Historical data may be utilized on a case-by-case basis. Historical data may include aggregated statistics of many people that relate to the current procedure, patient, and/or situation. Historical data may utilize data from within the same surgery.

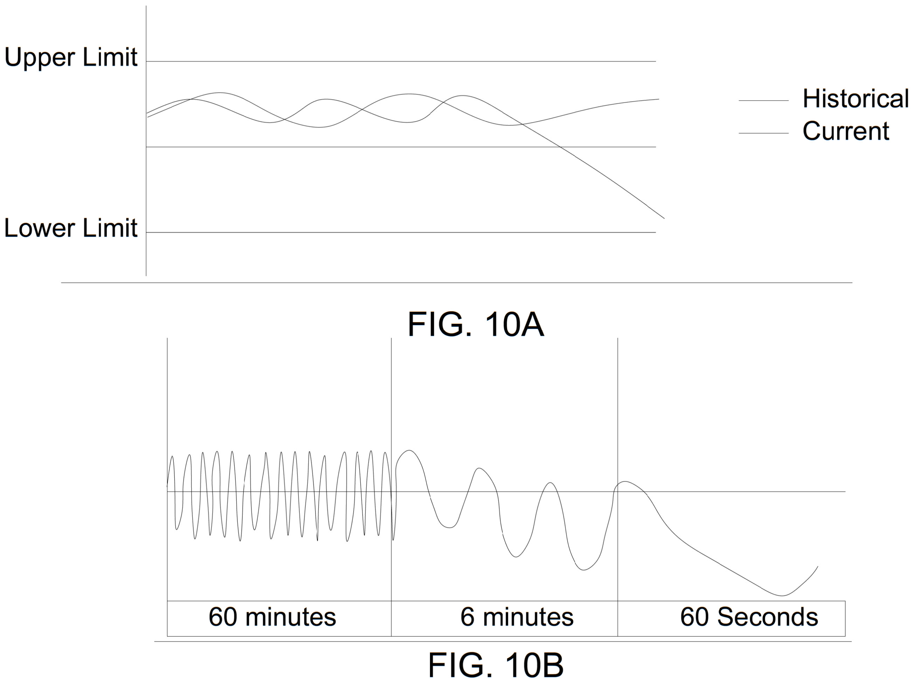

As shown in FIG. 10A, the system may display the historical data overlaid on current data. This may allow the user to quickly visualize discrepancies from the expected data values (e.g., such as the sudden drop in FIG. 10A). Expected data may be defined as data that fits within the limits or physiological ranges for the human body or within the operable ranges of equipment.

The presentation of data may be simplified. For example, the presentation of historical data may be simplified by presenting the data over a logarithmic axis with time. As shown in FIG. 10B, the system may show historical data from the previous 60 minutes, 6 minutes, and 60 seconds of the surgical procedure. In this case, the historical data may show minimums, maximums, and/or averages collapsed relative to time (e.g., so that all data can be shown within a single snapshot). For example, in FIG. 10B, the 60-minute segment appears relatively consistent, the 6-minute segment shows a gradual decline, and the 60-second segment shows a more rapid decline. The system may use waterfall graphs to display data.

The surgeon may manually verify data readings from a sensor (e.g., based on a secondary reading performed by the surgeon). The system may indicate potential locations for sensor applications. For example, if a sensor was placed in the wrong or a subpar location, the system may indicate other (e.g., better) locations to apply the sensor.

The system may propose correction(s) and/or procedure step change(s) based on a data stream. Multiple signals impacting the same control loop may lead the system toward different decisions. If the system cannot reconcile the differences to make an automated decision, the system may seek surgeon input.

The system may use cascading warnings to warn the user. For example, the system may show system interconnections at which the issue cascades from one system to the next.

A first smart system may notify a second smart system that it may be likely to interfere or impact a measurement aspect of the second smart system. The first smart system may sequentially cascade the warnings/notifications to the other systems that are downstream dependent on those monitored aspects (e.g., for closed loop control or transformations).

The system may warn subsequent systems that the data will be affected or missing. The system may supply a replacement value for the missing data. The system may indicate a duration of the effected aspect (e.g., a length of time during which the replacement value is to be used).

The duration of, intensity of, or reaction to the warning may intensify (e.g., based on the issue). For example, if a temperature sensor on a patient warming device is faulty (e.g., the function of the patient warming device to warm the patient is disabled), the patient risk of hypothermia may increase. The system may warn the surgeon of the change. The system may cascade the warning from the patient warming device to the patient transcutaneous temperature sensor or smart ventilator. The warning may let the other devices know that readings and/or patient condition may (e.g., are likely to) change.

The system may output discrete notifications. The smart system (e.g., acting on data feed from another system) may detect that the feed violates one or more of the closed loop envelope operational window(s). The thresholds may be tiered with higher or lower levels (e.g., resulting in more intense notifications or actions as the level increases).

Example discrete electrical failures are described herein. A discrete electrical failure may include hardware failures, PTC resetting events, high signal noise (e.g., reduction of the signal-to-noise ratio), shorting events, unstable power lines/voltage rails, a brown out, a black out, fluctuations, and/or the like.

The system may output notifications related to continuous feed(s). The data stream may have a continuous feed or data. In some examples, the value of the data stream at a given time (e.g., after detection of the issue) may be as critical as the violation of the bounded thresholds. For example, the system may use the dynamic rate of reaction to adjust warnings/notifications (e.g., based on the value's closeness to threshold).

The system may establish a rate of warning escalation for a continuous feed. The threshold and/or rate may be adjustable based on the situation/user. For example, proximity detection of robotic arms may cause different levels of reaction (e.g., depending on tools attached to the arms).

The surgeon or system may adjust inputs based on bandwidth usage. For example, the system may be aware of the available bandwidth and its rate of consumption. The system may determine (e.g., based on other smart systems) whether to reduce its bandwidth consumption.

A physical workspace may share notifications. For example, the system may be aware of the physical constraints of itself and its workspace. The system may determine velocity limits, position limits, acceleration limits, etc. for the workspace.

Warning severity escalation may be in reaction to confusing data streams. The system may combine warnings/notifications from multiple data streams (e.g., to generate a more severe/elevated warning). For example, a visualization system may detect a bleeding event and send a notification to the supervisory smart system. Depending on the level of bleeding, the supervisory smart system may react differently (e.g., elevate the warning response to the user, send request messages to other smart systems to query for more surgical environmental information, etc.).

In another example, the smart system may identify an energy device being activated off screen. The system may send a warning to another smart system that the surgeon is purposefully activating the energy device. In this case, neither system may display the warning. The system may determine a course of action (e.g., system shutdown or notify a user of a warning).

The smart system may determine whether to notify the user based on a known or calculated accuracy of the data. The smart system may average or filter data (e.g., on the fly) to compensate for inaccuracies. The smart system may check multiple sources to understand variations in the data (e.g., to better compensate for the inaccuracies).

Event timing may be used to resolved conflicting data. If the timing of an event is critical, the system may decide to present a warning. The system may pause notification to prevent unwanted movement during critical activation. For example, the system may lock out movement while stapling or sealing. A harmonic device may be allowed to proceed with movement to allow for cutting. During startup, the system may determine that all devices are booted and idle prior to continuing the procedure.

Multiple simultaneous devices may be activated from multiple independent generators. A capacitive grounding pad may be shared by multiple monopolar generators.

Example 1. A surgical system, comprising:
a processor configured to:
receive a first biomarker value associated with a first biomarker in a first data stream and a second biomarker value associated a second biomarker in a second data stream;
determine, based on the first biomarker value and the second biomarker value, that a close-loop control condition associated with a control parameter for a surgical device is satisfied;
based on determining that the close-loop control condition is satisfied, determine a control parameter value associated with the surgical device based on the first biomarker value and the second biomarker value;
generate a control signal for the surgical device based on the determined control parameter value;
receive a third biomarker value associated with the first biomarker in the first data stream and a fourth biomarker value associated with the second biomarker in the second data stream;
determine, based on the third biomarker value and the fourth biomarker value, that the close-loop control condition associated with the control parameter for the surgical device is failed;
based on determining that the close-loop control condition is failed, identify an intraoperative metric associated with the first data stream and the second data stream; and
generate a second control signal configured to display a value associated with the intraoperative metric.

Example 2. The surgical system of example 1, wherein the first biomarker and the second biomarker are associated with a physiological function of a patient, and the processor is further configured to:
determine a first status of the physiological function based on the first biomarker value and the second biomarker value, wherein the close-loop control condition is determined to be satisfied based on the first status of the physiological function being within an expected range; and
determine a second status of the physiological function based on the third biomarker value and the fourth biomarker value, wherein the close-loop control condition is determined to be failed based on the second status of the physiological function being outside the expected range.

Example 3. The surgical system of example 1 or 2, wherein the processor is further configured to determine a status type of the second status, wherein the status type indicates at least one of:
at least one of the first biomarker or the second biomarker has changed at a rate that is greater than a first threshold,
at least one of the first biomarker or the second biomarker has fluctuated a number of times during a time window, wherein the number of times is greater than a second threshold ,
a difference between the first biomarker and the second biomarker is greater than a third threshold, or
a timing delay between a change in the first data stream and a change in the second data stream is greater than a fourth threshold, wherein the intraoperative metric is identified based on the status type of the second status.

Example 4. The surgical system of any one of examples 1-3, wherein the first biomarker and the second biomarker are associated with a physiological function of a patient, the processor is further configured to:
identify a third biomarker associated with the physiological function of the patient;
determine that the third biomarker is capable of impacting at least one of the first biomarker or the second biomarker ; and
based on the determination that the third biomarker is capable of impacting at least one of the first biomarker or the second biomarker, use the third biomarker as the intraoperative metric.

Example 5. The surgical system of any one of examples 1-4, wherein the processor is further configured to:
determine a first control parameter change direction associated with the control parameter based on the first biomarker value;
determine a second control parameter change direction associated with control parameter based on the second biomarker value, wherein the close-loop control condition is determined to be satisfied based on the first control parameter change direction and the second control parameter change direction being the same;
determine a third control parameter change direction associated with the control parameter based on the third biomarker value; and
determine a fourth control parameter change direction associated with the control parameter based on the fourth biomarker value, wherein the close-loop control condition is determined to be failed based on the third control parameter change direction and the fourth control parameter change direction being different.

Example 6. The surgical system of any one of examples 1-5, wherein the processor is further configured to:
determine a correlation pattern of the first data stream and the second data stream, wherein the close-loop control condition is determined to be satisfied or failed based on the correlation pattern.

Example 7. The surgical system of any one of examples 1-6, wherein the first biomarker is a blood oxygen content, the second biomarker is a percentage of carbon dioxide in exhalations, and the processor is further configured to:
determine a correlation pattern of blood oxygen content measurements in the first data stream and percentage of carbon dioxide in exhalations measurements in the second data stream, wherein the close-loop control condition is determined to be satisfied based on the correlation pattern indicating that the percentage of carbon dioxide in exhalations measurements and the blood oxygen content measurements change at a same rate; and
generate a visual indication of a slope comparison of the first data stream and the second data stream, wherein the intraoperative metric comprises the slope comparison of the first data stream and the second data stream.

Example 8. The surgical system of any one of examples 1-7, wherein the first biomarker is a blood oxygen content, the second biomarker is a percentage of carbon dioxide in exhalations, and the processor is further configured to:
determine a correlation pattern of blood oxygen content measurements in the first data stream and percentage of carbon dioxide in exhalations measurements in the second data stream, wherein the close-loop control condition is determined to be failed based on the correlation pattern indicating that the percentage of carbon dioxide in exhalations measurements and the blood oxygen content measurements drift apart; and
based on determining that the percentage of carbon dioxide in exhalations measurements and the blood oxygen content measurements drift apart, identify a core body temperature of a patient as the intraoperative metric for display.

Example 9. The surgical system of any one of examples 1-8, wherein the processor is further configured to:
determine a first pattern of the first data stream; and
determine a second pattern of the second data stream, wherein the intraoperative metric comprises the first pattern of the first data stream and the second pattern of the second data stream.

Example 10. The surgical system of any one of examples 1-9, wherein the processor is further configured to:
determine a timing delay between a change in the first data stream and a change in the second data stream, wherein the intraoperative metric comprises the determined timing delay between the change in the first data stream and the change in the second data stream.

Example 11. The surgical system of any one of examples 1-10, wherein the processor is further configured to:
determine that the first data stream has stopped being received; and
based on determining that the first data stream has stopped, include, in the intraoperative metric comprises an option to use simulated data based on a pattern of the first biomarker while the first data stream was being received.

Example 12. The surgical system of any one of examples 1-11, wherein the processor is further configured to:
determine a format of a graphical representation of the first data stream and the second data stream based on the intraoperative metric ; and
generate the graphical representation based on the determined format, wherein the second control signal is configured to instruct a display to display the generated graphical representation.

Example 13. The surgical system of any one of examples 1-12, wherein the second control signal is further configured to indicate a prompt or suggestion, and the processor is further configured to:
receive an input in response to the prompt or suggestion; and
generate a third control signal for the surgical device based on received response.

Example 14. A method, performed by a surgical system, the method comprising:
receiving a first biomarker value associated with a first biomarker in a first data stream and a second biomarker value associated a second biomarker in a second data stream;
determining, based on the first biomarker value and the second biomarker value, that a close-loop control condition associated with a control parameter for a surgical device is satisfied;
based on determining that the close-loop control condition is satisfied, determining a control parameter value associated with the surgical device based on the first biomarker value and the second biomarker value;
generating a control signal for the surgical device based on the determined control parameter value;
receiving a third biomarker value associated with the first biomarker in the first data stream and a fourth biomarker value associated with the second biomarker in the second data stream;
determining, based on the third biomarker value and the fourth biomarker value, that the close-loop control condition associated with the control parameter for the surgical device is failed;
based on determining that the close-loop control condition is failed, identifying an intraoperative metric associated with the first data stream and the second data stream; and
generating a second control signal configured to display a value associated with the intraoperative metric.

Example 15. The method of example 14, wherein the first biomarker and the second biomarker are associated with a physiological function of a patient, and the method further comprises:
determining a first status of the physiological function based on the first biomarker value and the second biomarker value, wherein the close-loop control condition is determined to be satisfied based on the first status of the physiological function being within an expected range; and
determining a second status of the physiological function based on the third biomarker value and the fourth biomarker value, wherein the close-loop control condition is determined to be failed based on the second status of the physiological function being outside the expected range.

Example 16. The method of example 14 or 15, wherein the method further comprises determining a status type of the second status, wherein the status type indicates at least one of:
at least one of the first biomarker or the second biomarker has changed at a rate that is greater than a first threshold,
at least one of the first biomarker or the second biomarker has fluctuated a number of times during a time window, wherein the number of times is greater than a second threshold ,
a difference between the first biomarker and the second biomarker is greater than a third threshold, or
a timing delay between a change in the first data stream and a change in the second data stream is greater than a fourth threshold, wherein the intraoperative metric is identified based on the status type of the second status.

Example 17. The method of any one of examples 14-16, wherein the first biomarker and the second biomarker are associated with a physiological function of a patient, the method further comprises:
identifying a third biomarker associated with the physiological function of the patient;
determining that the third biomarker is capable of impacting at least one of the first biomarker or the second biomarker ; and
based on the determination that the third biomarker is capable of impacting at least one of the first biomarker or the second biomarker, using the third biomarker as the intraoperative metric.

Example 18. The method of any one of examples 14-17, wherein the method further comprises:
determining a first control parameter change direction associated with the control parameter based on the first biomarker value;
determining a second control parameter change direction associated with control parameter based on the second biomarker value, wherein the close-loop control condition is determined to be satisfied based on the first control parameter change direction and the second control parameter change direction being the same;
determining a third control parameter change direction associated with the control parameter based on the third biomarker value; and
determining a fourth control parameter change direction associated with the control parameter based on the fourth biomarker value, wherein the close-loop control condition is determined to be failed based on the third control parameter change direction and the fourth control parameter change direction being different.

Example 19. The method of any one of examples 14-18, wherein the method further comprises:
determining a correlation pattern of the first data stream and the second data stream, wherein the close-loop control condition is determined to be satisfied or failed based on the correlation pattern.

Example 20. The method of any one of examples 14-19, wherein the first biomarker is a blood oxygen content, the second biomarker is a percentage of carbon dioxide in exhalations, and the method further comprises:
determining a correlation pattern of blood oxygen content measurements in the first data stream and percentage of carbon dioxide in exhalations measurements in the second data stream, wherein the close-loop control condition is determined to be satisfied based on the correlation pattern indicating that the percentage of carbon dioxide in exhalations measurements and the blood oxygen content measurements change at a same rate; and
generating a visual indication of a slope comparison of the first data stream and the second data stream, wherein the intraoperative metric comprises the slope comparison of the first data stream and the second data stream.

Although features and elements are described above in particular combinations, one of ordinary skill in the art will appreciate that each feature or element can be used alone or in any combination with the other features and elements. In addition, the methods described herein may be implemented in a computer program, software, or firmware incorporated in a computer-readable medium for execution by a computer or processor. Examples of computer-readable media include electronic signals (transmitted over wired or wireless connections) and computer-readable storage media. Examples of computer-readable storage media include, but are not limited to, a read only memory (ROM), a random access memory (RAM), a register, cache memory, semiconductor memory devices, magnetic media such as internal hard disks and removable disks, magnetooptical media, and optical media such as CD-ROM disks, and digital versatile disks (DVDs). A processor in association with software may be used to implement a radio frequency transceiver for use in a WTRU, UE, terminal, base station, RNC, or any host computer.

## Claims

1. A surgical system comprising a processor configured to:
receive a first biomarker value associated with a first biomarker in a first data stream and a second biomarker value associated a second biomarker in a second data stream;
determine, based on the first biomarker value and the second biomarker value, that a close-loop control condition associated with a control parameter for a surgical device is satisfied;
based on determining that the close-loop control condition is satisfied, determine a control parameter value associated with the surgical device based on the first biomarker value and the second biomarker value;
generate a control signal for the surgical device based on the determined control parameter value;
receive a third biomarker value associated with the first biomarker in the first data stream and a fourth biomarker value associated with the second biomarker in the second data stream;
determine, based on the third biomarker value and the fourth biomarker value, that the close-loop control condition associated with the control parameter for the surgical device is failed;
based on determining that the close-loop control condition is failed, identify an intraoperative metric associated with the first data stream and the second data stream; and
generate a second control signal configured to display a value associated with the intraoperative metric.

2. The surgical system of claim 1, wherein the first biomarker and the second biomarker are associated with a physiological function of a patient, and the processor is further configured to:
determine a first status of the physiological function based on the first biomarker value and the second biomarker value, wherein the close-loop control condition is determined to be satisfied based on the first status of the physiological function being within an expected range; and
determine a second status of the physiological function based on the third biomarker value and the fourth biomarker value, wherein the close-loop control condition is determined to be failed based on the second status of the physiological function being outside the expected range,
optionally wherein the processor is further configured to determine a status type of the second status, wherein the status type indicates at least one of:
at least one of the first biomarker or the second biomarker has changed at a rate that is greater than a first threshold,
at least one of the first biomarker or the second biomarker has fluctuated a number of times during a time window, wherein the number of times is greater than a second threshold ,
a difference between the first biomarker and the second biomarker is greater than a third threshold, or
a timing delay between a change in the first data stream and a change in the second data stream is greater than a fourth threshold, wherein the intraoperative metric is identified based on the status type of the second status.

3. The surgical system of claim 1 or claim 2, wherein the first biomarker and the second biomarker are associated with a physiological function of a patient, the processor is further configured to:
identify a third biomarker associated with the physiological function of the patient;
determine that the third biomarker is capable of impacting at least one of the first biomarker or the second biomarker ; and
based on the determination that the third biomarker is capable of impacting at least one of the first biomarker or the second biomarker, use the third biomarker as the intraoperative metric.

4. The surgical system of any one of the preceding claims, wherein the processor is further configured to:
determine a first control parameter change direction associated with the control parameter based on the first biomarker value;
determine a second control parameter change direction associated with control parameter based on the second biomarker value, wherein the close-loop control condition is determined to be satisfied based on the first control parameter change direction and the second control parameter change direction being the same;
determine a third control parameter change direction associated with the control parameter based on the third biomarker value; and
determine a fourth control parameter change direction associated with the control parameter based on the fourth biomarker value, wherein the close-loop control condition is determined to be failed based on the third control parameter change direction and the fourth control parameter change direction being different.

5. The surgical system of any one of the preceding claims, wherein the processor is further configured to:
determine a correlation pattern of the first data stream and the second data stream, wherein the close-loop control condition is determined to be satisfied or failed based on the correlation pattern.

6. The surgical system of any one of the preceding claims, wherein the first biomarker is a blood oxygen content, the second biomarker is a percentage of carbon dioxide in exhalations, and the processor is further configured to:
determine a correlation pattern of blood oxygen content measurements in the first data stream and percentage of carbon dioxide in exhalations measurements in the second data stream, wherein the close-loop control condition is determined to be satisfied based on the correlation pattern indicating that the percentage of carbon dioxide in exhalations measurements and the blood oxygen content measurements change at a same rate; and
generate a visual indication of a slope comparison of the first data stream and the second data stream, wherein the intraoperative metric comprises the slope comparison of the first data stream and the second data stream.

7. The surgical system of any one of the preceding claims, wherein the first biomarker is a blood oxygen content, the second biomarker is a percentage of carbon dioxide in exhalations, and the processor is further configured to:
determine a correlation pattern of blood oxygen content measurements in the first data stream and percentage of carbon dioxide in exhalations measurements in the second data stream, wherein the close-loop control condition is determined to be failed based on the correlation pattern indicating that the percentage of carbon dioxide in exhalations measurements and the blood oxygen content measurements drift apart; and
based on determining that the percentage of carbon dioxide in exhalations measurements and the blood oxygen content measurements drift apart, identify a core body temperature of a patient as the intraoperative metric for display.

8. The surgical system of any one of the preceding claims, wherein the processor is further configured to:
determine a first pattern of the first data stream; and
determine a second pattern of the second data stream, wherein the intraoperative metric comprises the first pattern of the first data stream and the second pattern of the second data stream.

9. The surgical system of any one of the preceding claims, wherein the processor is further configured to:
determine a timing delay between a change in the first data stream and a change in the second data stream, wherein the intraoperative metric comprises the determined timing delay between the change in the first data stream and the change in the second data stream.

10. The surgical system of any one of the preceding claims, wherein the processor is further configured to:
determine that the first data stream has stopped being received; and
based on determining that the first data stream has stopped, include, in the intraoperative metric comprises an option to use simulated data based on a pattern of the first biomarker while the first data stream was being received.

11. The surgical system of any one of the preceding claims, wherein the processor is further configured to:
determine a format of a graphical representation of the first data stream and the second data stream based on the intraoperative metric ; and
generate the graphical representation based on the determined format, wherein the second control signal is configured to instruct a display to display the generated graphical representation.

12. The surgical system of any one of the preceding claims, wherein the second control signal is further configured to indicate a prompt or suggestion, and the processor is further configured to:
receive an input in response to the prompt or suggestion; and
generate a third control signal for the surgical device based on received response.

13. A method, performed by a surgical system, the method comprising:
receiving a first biomarker value associated with a first biomarker in a first data stream and a second biomarker value associated a second biomarker in a second data stream;
determining, based on the first biomarker value and the second biomarker value, that a close-loop control condition associated with a control parameter for a surgical device is satisfied;
based on determining that the close-loop control condition is satisfied, determining a control parameter value associated with the surgical device based on the first biomarker value and the second biomarker value;
generating a control signal for the surgical device based on the determined control parameter value;
receiving a third biomarker value associated with the first biomarker in the first data stream and a fourth biomarker value associated with the second biomarker in the second data stream;
determining, based on the third biomarker value and the fourth biomarker value, that the close-loop control condition associated with the control parameter for the surgical device is failed;
based on determining that the close-loop control condition is failed, identifying an intraoperative metric associated with the first data stream and the second data stream; and
generating a second control signal configured to display a value associated with the intraoperative metric.

14. The method of claim 13, wherein the first biomarker and the second biomarker are associated with a physiological function of a patient, and the method further comprises:
determining a first status of the physiological function based on the first biomarker value and the second biomarker value, wherein the close-loop control condition is determined to be satisfied based on the first status of the physiological function being within an expected range; and
determining a second status of the physiological function based on the third biomarker value and the fourth biomarker value, wherein the close-loop control condition is determined to be failed based on the second status of the physiological function being outside the expected range,
optionally wherein the method further comprises determining a status type of the second status, wherein the status type indicates at least one of:
at least one of the first biomarker or the second biomarker has changed at a rate that is greater than a first threshold,
at least one of the first biomarker or the second biomarker has fluctuated a number of times during a time window, wherein the number of times is greater than a second threshold ,
a difference between the first biomarker and the second biomarker is greater than a third threshold, or
a timing delay between a change in the first data stream and a change in the second data stream is greater than a fourth threshold, wherein the intraoperative metric is identified based on the status type of the second status.

15. The method of claim 13 or claim 14, wherein the first biomarker and the second biomarker are associated with a physiological function of a patient, the method further comprises:
identifying a third biomarker associated with the physiological function of the patient;
determining that the third biomarker is capable of impacting at least one of the first biomarker or the second biomarker ; and
based on the determination that the third biomarker is capable of impacting at least one of the first biomarker or the second biomarker, using the third biomarker as the intraoperative metric.

16. The method of any one of claims 13 to 15, wherein the method further comprises:
determining a first control parameter change direction associated with the control parameter based on the first biomarker value;
determining a second control parameter change direction associated with control parameter based on the second biomarker value, wherein the close-loop control condition is determined to be satisfied based on the first control parameter change direction and the second control parameter change direction being the same;
determining a third control parameter change direction associated with the control parameter based on the third biomarker value; and
determining a fourth control parameter change direction associated with the control parameter based on the fourth biomarker value, wherein the close-loop control condition is determined to be failed based on the third control parameter change direction and the fourth control parameter change direction being different.

17. The method of claim any one of claims 13 to 16, wherein the method further comprises:
determining a correlation pattern of the first data stream and the second data stream, wherein the close-loop control condition is determined to be satisfied or failed based on the correlation pattern.

18. The method of claim any one of claims 13 to 17, wherein the first biomarker is a blood oxygen content, the second biomarker is a percentage of carbon dioxide in exhalations, and the method further comprises:
determining a correlation pattern of blood oxygen content measurements in the first data stream and percentage of carbon dioxide in exhalations measurements in the second data stream, wherein the close-loop control condition is determined to be satisfied based on the correlation pattern indicating that the percentage of carbon dioxide in exhalations measurements and the blood oxygen content measurements change at a same rate; and
generating a visual indication of a slope comparison of the first data stream and the second data stream, wherein the intraoperative metric comprises the slope comparison of the first data stream and the second data stream.

19. A computer-readable medium comprising instructions which, when executed by a processor, cause the processor to carry out the method of any one of claims 13 to 18.
